# EUROPEAN PATENT APPLICATION

(11) **EP 0 664 101 A1**
(43) Date of publication of application: **26.07.1995**
(21) Application number: 94300469.7
(22) Date of filing: 21.01.1994
(51) Int. Cl.: A61B 1/12

(54) **Lens cleaning apparatus**

(71) Applicant: DEVMED GROUP INC., Palm Beach Gardens, FL 33410 (US)
(72) Inventor: Wiita, Bruce E., Dr., North Palm Beach, FL 33408, Florida (US); Teets, Michael J., Hobe Sound, FL 33455 (US); Wiita, Gregory D., Palm Beach Gardens, FL. 33410, Florida (US)
(74) Representative: Leale, Robin George

(57) **Abstract**

A hollow tubular member (10) is concentrically mounted to a borescope or surgical viewing instrument to define a spaced passage (22) for flowing fluid to a cuff (46) which is discretely located below the distal end of the lens (53) of the borescope and which defines a discretely configured discharge port (50) for flowing fluid over the lens surface for cleansing and defogging purposes. A two-piece locking handle (132,140) may be used to lock the lens cleaning apparatus to the borescope. Another embodiment includes a flexible tube (90) utilized with the cuff.

## Description

This invention relates to biological viewing instruments that include a lens to be associated with a video camera or the like for internal viewing of the body, and particularly to means for cleaning and shielding the lens of such an instrument.

There are a number of lens cleaning devices that are described in the prior art for use on endoscopes, resectoscopes, fiberscopes, catheterisation devices and the like, for cleaning a lens thereof. For example U.S. Patent 4,770,163 granted to Ono et al on September 13, 1988 and U.S. Patent No. 4,576,146 granted to Kawazoe et al on March 18, 1986 disclose apparatus for viewing blood vessels and the like whilst continuously flowing saline fluid through a passage formed in an endoscope at a flow rate that approximates the blood flow rate. Of particular interest is the lens cleaning means disclosed in these patents which show a pair of passageways that include outlets that flow the saline solution in front of the lens. The outlets serve to orient the flow so that the flows discharging from the outlets of the respective passageways oppose each other and hence, according to these patents, the interaction of the opposing fluid flows keeps the lens clean.

Also of interest as disclosed in the 4,576,146 patent, supra, is a spiral passageway for injecting saline solution with a spiral flow to displace opaque liquid in the region of observation.

U.S. Patent No. 4,690,140 granted to Mecca on September 1, 1987 discloses an endoscopic tube with a passageway to route clear liquid to circulate around the endoscopic tube. This is another attempt to keep the lens clean.

U.S. Patent No. 4,633,855 granted to Baba on January 6, 1987 also discloses an endoscope that includes a tube mounted internally within the endoscope and having its distal end bent at approximately 90 degrees and directed toward the lens so as to blow air or water adjacent the observation window in order to keep it clean.

While these lens cleaning means may be satisfactory in certain biological procedures, they are not satisfactory for others. In the application of video-surgery in laporoscopic and arthroscopic procedures, for example, we have found that causing a film of cleaning fluid to form over the lens surface is a far more satisfactory method of keeping the lens clean, or defogging it if it fogs up. In certain instances injection of the fluid, which may be water or carbon dioxide for example, intermittently as and when needed has proven to be a satisfactory method of keeping the lens clean and defogged.

Viewed from one aspect the present invention provides apparatus for cleaning the lens of a viewing camera used in biological observation by penetration into a cavity of a living being, the camera having an elongate tube housing fibre optics for producing a lighted area from a light source and an image relay for a television camera, the apparatus comprising:
a tubular member arranged to be concentrically mounted around said elongate tube to form a longitudinal passageway therewith,
a cuff portion arranged to extend beyond the distal end of said elongate tube to reduce lens contact with body parts and thus inhibit opaque fluids from obscuring visibility, said cuff portion including fluid passage means in fluid communication with said longitudinal passageway and defining at least one discharge port at the end of said elongate tube, said cuff portion including a bottom wall surface for redirecting the flow of fluid in said passage means to flow into said discharge port and flow laterally in a direction to scrub the surface of the lens mounted at the distal end of said elongate tube,
said discharge port being sized to coalesce and direct the flow of fluid to form a sheet of fluid, and
means for admitting fluid into said longitudinal passageway whereby the flow of fluid formed into a sheet may be directed over the lens on demand of the operator.

Viewed from another aspect the invention provides apparatus for cleaning the lens of a viewing camera used in biological observation by penetration into a cavity of a living being, the camera having an elongate tube housing fibre optics for producing a lighted area from a light source and an image relay for a television camera, with a lens at the distal end of said elongate tube, the apparatus comprising:
a tubular member concentrically mounted around said elongate tube and being spaced therefrom to form a longitudinal passageway for cleansing fluid to flow to said lens,
means for generating a vortex of said fluid disposed at the end of said longitudinal passageway, said means for generating a vortex including a plurality of vanes circumferentially spaced about an axis that is disposed coaxially relative to the longitudinal axis of said lens, and
means for admitting fluid into said longitudinal passageway whereby the flow of fluid formed into a vortex may be directed over the lens on demand of an operator.

Thus in some embodiments this invention employs a radial cavity with either partial or full circumferential flow directed over the surface of a lens or lens cover. In other embodiments the invention contemplates incorporating a vortex generator circumferentially mounted around the lens. The invention also contemplates incorporating a fluid conveying passage within a flexible tube for surrounding flexible types of medical instruments.

A further preferred feature of the invention is to provide a tubular member surrounding the tube supporting the fibre optics that provide light and transmit the image to a camera (video or otherwise) used in video surgery, which tubular member includes a passage for flowing fluid to the discharge end, and a radial channel with partial or full fluid flow capability formed in a cuff downstream of the lens, and means to redirect the flow and coalesce the fluid and direct a film of fluid adjacent the surface and toward the center of the lens.

A further preferred feature of the invention is to provide apparatus incorporating a tubular member with means for cleaning and/or shielding the lens of a biological observation instrument and which is capable of being adapted to existing instruments or being fabricated integrally with such instruments. Such apparatus can be sterilized by an autoclave method. The apparatus can also be used to irrigate an adjacent area in the body by flowing fluid through the same, to obtain a clearer view of the surrounding area.

Another preferred feature of the invention is the incorporation of a two-part handle having a lower part connected to the lens cleaning apparatus and an upper part rotatable relative to the lower part and the parts having a camming arrangement for locking the lens carrying tube by a slight turn of the one handle part relative to the other.

Some embodiments of the invention will now be described by way of example and with reference to the accompanying drawings, in which:-
FIG. 1 is a perspective view partly in section illustrating an embodiment of the invention adapted to fit a borescope,
FIG. 2 is an enlarged partial view in section showing details of the lens cleaning passages of the embodiment shown in FIG. 1,
FIG. 3 is an enlarged sectional view of the end cap for securing the embodiment to an existing borescope,
FIG. 4 is an enlarged partial sectional view illustrating another embodiment of the invention,
FIG. 5 is an enlarged view illustrating a vortex generator attachment,
FIG. 6 is an enlarged partial sectional view illustrating another embodiment of the invention,
FIG. 7 is a partial view in section taken along line 7-7 of FIG. 6,
FIG. 8 is a view in perspective illustrating another embodiment of the invention when applied to a flexible borescope,
FIG. 8A is a partial view in section of the FIG. 8 embodiment, illustrating a rib portion for defining a helical flow path,
FIG. 9 is a partial view in perspective and partly in section illustrating an embodiment of the invention employing a valving arrangement for controlling the cleaning fluid,
FIG. 10 is a partial view in section showing details of the embodiment of FIG. 9,
FIG. 11 is an exploded view in perspective illustrating a two-part locking handle,
FIG. 12 is a partial view partly in section illustrating the two-part piece locking handle, and
FIG. 13 is a sectional view, partly in phantom, taken along line 13-13 of FIG. 12 and illustrating the handle in the unlocked position.

While in its preferred embodiments this invention is intended for use in video surgery, as for example for performing appendectomies, removal of gall bladders, removal of cancerous prostrate glands, and for biological observation, it is to be understood that the invention has application in any circumstances where a lens is to be inserted into a cavity and ready access to clean the lens is not available. In a typical medical operation using view cameras the portion of the body to be observed is invaded by a Trocar which incises a small cylindrical hole through the body skin and tissue and is then withdrawn leaving a hollow plastics tube in place. A tubular instrument (hereinafter referred to as a borescope) is then inserted which carries bundles of fiber optics which serve to transmit high intensity light beams to illuminate the area being treated and to carry images back to a TV camera to view the sighted area. The present invention is concerned with cleaning and shielding the lens that is located at the distal end of the borescope.

While the invention mainly contemplates providing cleaning and shielding means for use with existing borescopes, it is also within the scope of the invention to provide a borescope already incorporating such means.

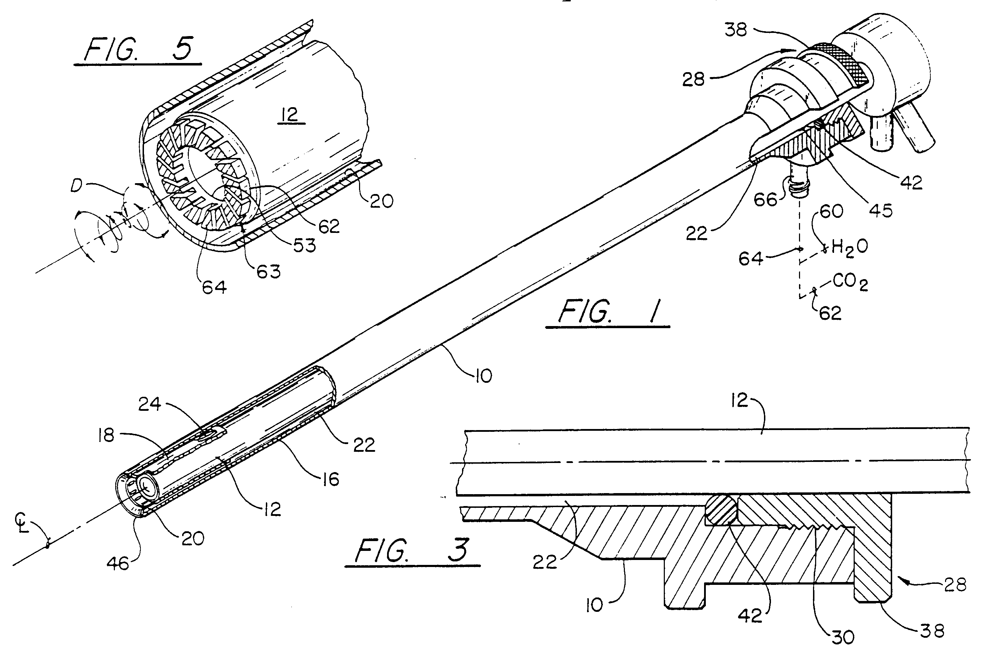

To best understand this invention reference is now made to FIGS. 1-3 depicting a hollow cylindrical tube 10 adapted to concentrically fit over the borescope 12. The diameter is selected to provide an annular space between the outside diameter of the borescope 12 and the inner diameter of the tube 10 having specific dimensions for the passage of sufficient fluid necessary for lens cleansing and shielding.

In the preferred embodiment the bottom or distal end is formed in two cylindrical pieces concentrically mounted relative to each other. The outer piece or sheath 16 extends the entire length of the tube and the inner piece is a short tube 18 mounted at the distal end and extends axially a short distance up the sheath 16. The diameter of the inner short tube 18 forms a snug fit with the outer surface of the borescope 12 and is slightly spaced to form the annular passage 20 that is in fluid communication with the annular passage 22 defined by the sheath 16. A plurality of spacers 24 are circumferentially spaced and extend between short tube 18 and the inner surface of sheath 16 and are suitably bonded thereto and serve to keep the two tubes in concentric alignment.

A retention nut and gland combination 28 threadably engages threads 30 formed on the inner diameter of sheath 16 at the proximal end and serves to secure and seal the sheath to the borescope. The outer circumference at the end of nut 38 may be knurled in order to manually torque the nut to the sheath. Sealing means, say O-ring seal 42, seals off the end to the passage 22.

Fluid is supplied to passage 22 through opening 45 formed in the sheath 16 and fluid such as water or carbon dioxide from a source (not shown) is regulated by suitable and commercially available valves, such as trumpet valves that are normally biased close and merely require depressing the valve stem to open, similar to those used in musical instruments.

According to this embodiment, the fluid in passages 22 and 20 is directed to a cuff 46 extending from and forming a part of sheath 16 that protrudes beyond the distal end of borescope 12. The passage 20 in cuff 46 includes a curved or flat bottom end 47 that serves to change the direction of the flow in passage 20. By virtue of the momentum of the flow the stream is directed to flow in the transverse passages 50 and discharge through the annular spaced outlets 52 and is directed toward the central axis of the lens. The stream of fluid is made to coalesce to form a film or sheet of fluid to flow over the outer surface of the lens cover 53. The dimensions of passage 50 and annular outlet are critical in that they are sized to give direction and coalesce the flow into a film which serves to clean the lens cover or lens directly if no cover is utilized. The dimensions of the length of the transverse passage 50 depicted by arrow A and the height of transverse passage 50 depicted by arrow B are critical and are selected so that the size of dimension A is substantially larger than the size of dimension B. In actual tests satisfactory results were obtained when the dimension of A was twice the dimension of B.

In operation suitable valves 60 or 62 (schematically shown) are activated to flow fluid through the line 64 coupled to fitting 66 which admits the fluid to annular passages 22 and 20 via the opening 45. The flow proceeds to the cuff 46 where it is guided by the bottom surface 47 and is forced to redirect the flow into the plurality of circumferential spaced passages 50. The flow discharging from the passages 50 is directed toward the central axis CL of the borescope 12. The fluid may be preheated in order to accommodate the defogging feature of this embodiment.

Another advantage of the cuff 46 is that by virtue of the fact that it extends axially beyond the lens cover it serves to shield the lens from being in direct contact with body tissue and in fact creates a space between the outer edge of the cuff and the surface of the lens which allows the intensive light being transmitted by the fiber optics to diffuse in this space and maintain visibility.

As will be appreciated from the foregoing the array of outlets 52 is in a form of an annulus and is in proximity to the lens and in fact circumscribes the lens. This serves to attract, by capillary attraction, any liquid droplets that should remain on the lens after the liquid has been turned off. The attraction of the droplets is by an adhesion effect which has a tendency of drawing the liquid back toward the annulus and as a consequence the droplets are removed from the lens, thereby avoiding any distortion of the image being transmitted to the camera.

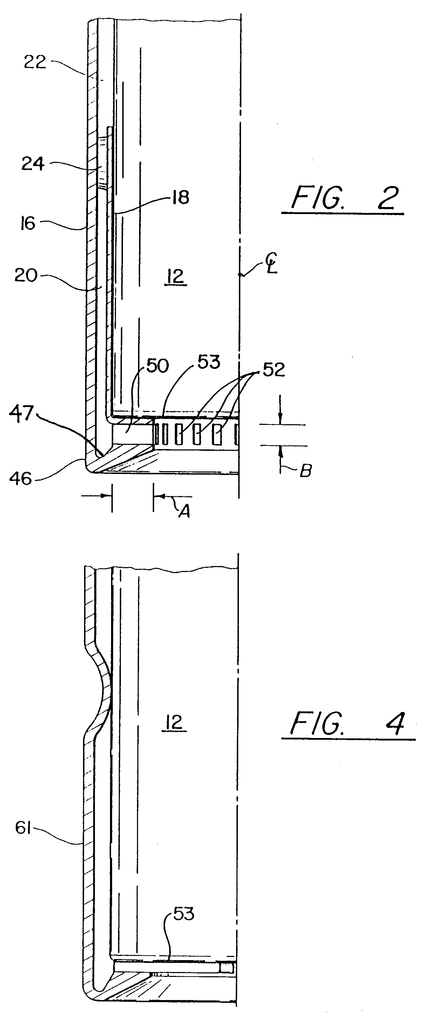

FIG. 4 depicts another embodiment of this invention where the number of outlets 52 are significantly reduced so that the span of the circumferential dimension is increased, simplifying the fabrication of the embodiment. Also the other sheath 61 is crimped at some distance up from the bottom end to form a dimple that bears against the outer circumferential surface of borescope 12 to keep the outer tube in concentric alignment. This replaces the spacers depicted in Fig. 1 to simplify the manufacture of the sheath.

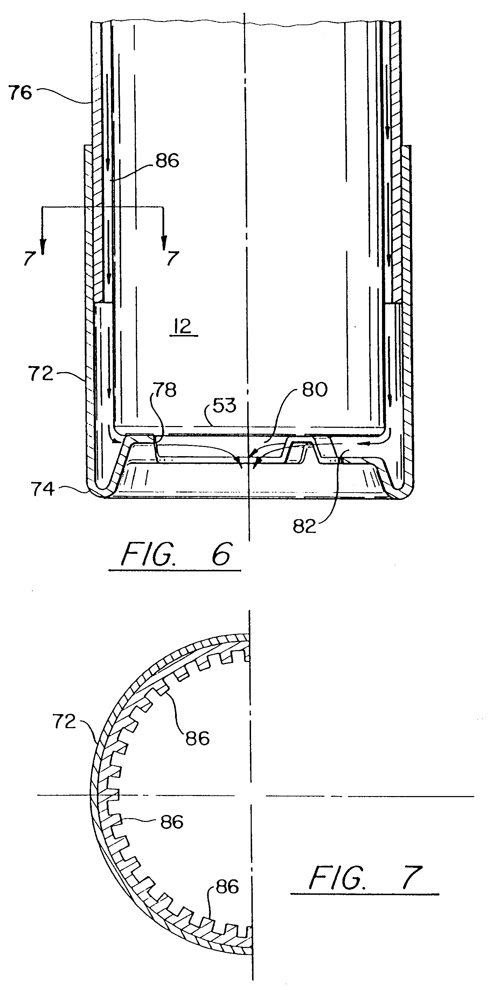

It is contemplated within the scope of this invention that instead of having the fluid discharge from the outlets 52 in a stream that is parallel or generally parallel to the lens cover, a vortex generator will be mounted at the outlet to impart a swirling motion to the fluid to in effect create vortices in front of the lens which will serve to not only clean the lens or its surface but also prevent loose tissue or other opaque substances from impinging on the lens. A suitable vortex generator generally indicated by reference numeral 63 is shown in Fig 5. Generally the vortex generator is a ring-like member 62 that carries a pluralities of upstanding vanes 64 spaced around the circumference. Vanes 64 serve to impart a swirl to the fluid passing therebetween so that the fluid discharging from the vanes defines a helical path depicted by the arrow D.

As is apparent from the foregoing the cleaning fluid that exits the channels defined between adjacent vanes 64 flows tangentially toward the center line CL, across the lens and with a spiral motion the fluid then travels in an axial/radial direction outwardly over the face of cuff 46 and as a secondary cleaning function serves to prevent opaque fluids present in the body from migrating from the cuff to the lens. The swirling fluid serves to not only keep the lens clean or defogged but also creates a shield to protect the lens from loose matter in the area being viewed.

The components of the lens cleaning system can be fabricated from suitable and well known metallic or non-metallic materials.

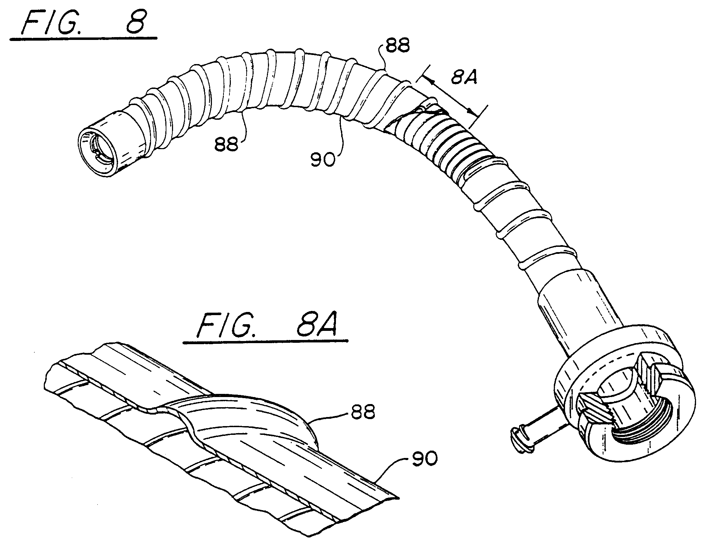

FIGS. 6 and 7 exemplify another embodiment of this invention where the short tube 72 defining cuff portion 74 is fabricated from sheet metal stock and is attached to the end of sheath 76 (similar to sheath 16 depicted in FIG. 1). The bottom end is crimped to define dimple 78. The lens cover 53 bears against the dimple 78 when installed and defines the space 80 to allow the flow discharging from the annular discharge 82 to flow over the surface of the lens. Similar to the embodiment depicted in FIG. 1, the cuff 74 serves to change the direction of the flow so that the flow passes beyond the lower extremity of the distal end of borescope 12 and is redirected to the outlet discharge end before being discharged adjacent the surface of the lens. In this embodiment of FIGS. 6 and 7 the tube 76 is held in concentric relationship with the borescope 12 by the scallops 86 formed on the inner diameter of tube 76.

FIGS. 8 and 8A exemplify this invention when the borescope is formed from a flexible tube. In this embodiment a helical formed channel 88 is formed in the flexible tube 90 which may be fabricated similar to BX metal cable to define the passage for flowing the fluid from the inlet to the discharge end. The cuff portion which can be identical to the versions shown in FIGS 1, 4, and 6 is secured to the end of the flexible tube 90. As seen in FIG. 8A the rib 88 defines a flexible helical channel to the boroscope outside diameter for delivering the fluid from the proximal end to the distal end of the lens cleaning tubular member.

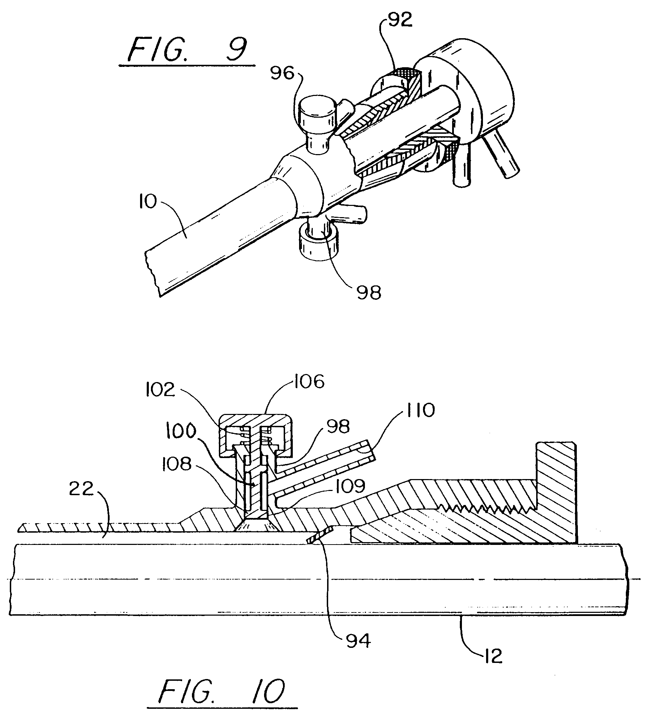

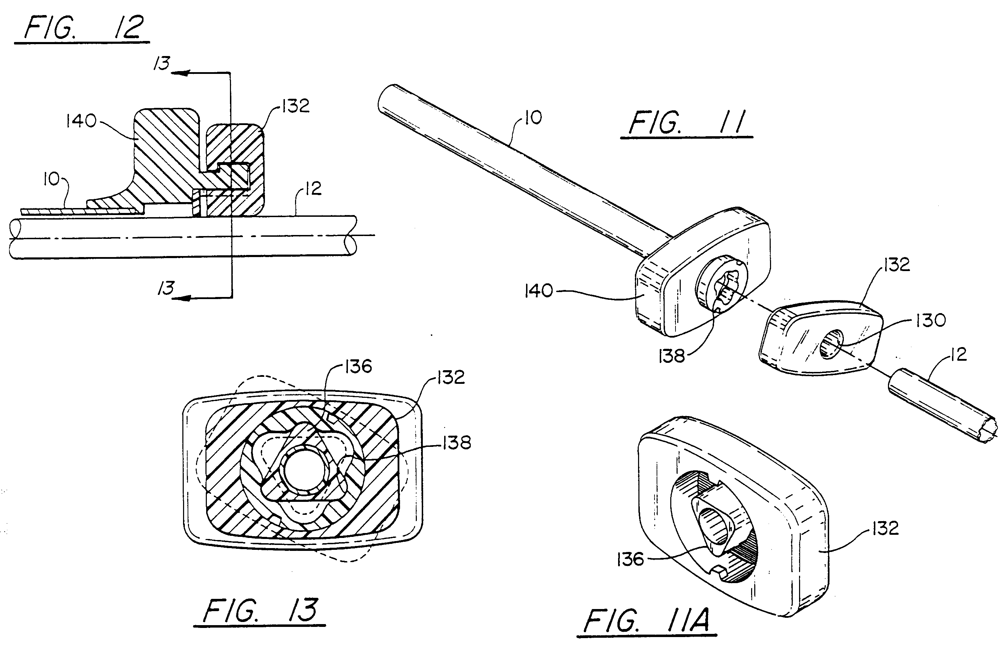

FIGS. 9 and 10 illustrate optional attachment means and trumpet valve means that can be employed with this invention. In this embodiment the borescope 12 is inserted in the tube 10 and held in place by end cap and bolt 92 threaded to the end of tube 10. A packing or gland illustrated by reference numeral 94 seals the end of the borescope 12. Suitable trumpet valves 96 and 98 serve to admit the fluid desired. The trumpet valve as shown in FIG. 10 consists of a plunger 100 that is spring biased by coil spring 102 in the upward direction. Depressing button 106 positions valve element 108 away from seat 109 placing passage 110 in fluid communication with passage 22.

In accordance with another feature and as can be seen in FIGS. 11 to 13 a two-piece handle is provided to conveniently lock the tubular member 10 to the borescope 12. The borescope 12 is inserted in the central passage 130 and extends throughout the distal end. The upper handle 132 carries an elongated triangular shaped cam element 136 that extends into the aperture 138 formed in lower handle 140. Aperture 138 is configured to define detents that have a smaller diameter area than the diameter area of the aperture 138. Hence, by rotating the upper handle 132 relative to the lower handle 140, which is held in place by the operator with the use of his other hand, the apex of the triangular shaped cam 136 fits into the smaller diameter area detents and the material of cam 136 is sufficiently flexible and resilient to frictionally engage the outer diameter of the borescope 12 and lock it into place.

As will be appreciated by those skilled in this art another use of the lens cleansing apparatus is the ability to irrigate the surrounding area by injecting a stream of fluid in the area where the surgeon requires a better or clearer view. To accomplish irrigation the operator merely depresses the trumpet valve to allow a stream of fluid to be injected in the surrounding area and leaves the valve in the operative mode until the area is visible. It is contemplated within the scope of this invention that the trumpet valve can include suitable means to hold the valve in the operative position.

## Claims

1. Apparatus for cleaning the lens (53) of a viewing camera used in biological observation by penetration into a cavity of a living being, the camera having an elongate tube (12) housing fibre optics for producing a lighted area from a light source and an image relay for a television camera, the apparatus comprising:
a tubular member (10) arranged to be concentrically mounted around said elongate tube to form a longitudinal passageway (22) therewith,
a cuff portion (46) arranged to extend beyond the distal end of said elongate tube to reduce lens contact with body parts and thus inhibit opaque fluids from obscuring visibility, said cuff portion including fluid passage means (20) in fluid communication with said longitudinal passageway and defining at least one discharge port (50) at the end of said elongate tube, said cuff portion including a bottom wall surface (47) for redirecting the flow of fluid in said passage means to flow into said discharge port and flow laterally in a direction to scrub the surface of the lens mounted at the distal end of said elongate tube,
said discharge port being sized to coalesce and direct the flow of fluid to form a sheet of fluid, and
means (60,62) for admitting fluid into said longitudinal passageway whereby the flow of fluid formed into a sheet may be directed over the lens on demand of the operator.

2. Apparatus as claimed in claim 1, wherein said discharge port (50) has a length (A) and height (B) and said length is larger than said height.

3. Apparatus as claimed in claim 3, wherein said length (A) is substantially equal to two times said height (B).

4. Apparatus as claimed in any preceding claim, wherein said longitudinal passageway (22) is annular.

5. Apparatus as claimed in any of claims 1 to 4, wherein said elongate tube (12) and said tubular member (90) are flexible and said longitudinal passageway is a helical passageway (88) formed by a helical rib portion of said tubular member.

6. Apparatus as claimed in any preceding claim, including a circumferential rim defined by said cuff (46) disposed relative to the edge of said discharge port (50) for adhering through capillary attraction droplets of fluid that remain in proximity to said lens (53) after the flow of fluid is discontinued.

7. Apparatus as claimed in any preceding claim, including means (63) for generating a vortex of said fluid disposed at the end of said discharge port.

8. Apparatus as claimed in claim 7, wherein said means for generating a vortex includes a plurality of spaced vanes (64) circumferentially spaced about an axis (CL) that is arranged to be disposed coaxially relative to the longitudinal axis of said lens (53).

9. Apparatus as claimed in any preceding claim, wherein the internal diameter of said tubular member (10) is threaded, an end cap (28) including a threaded shank portion (30) being threadably supported to said internal threads for placing said elongate tube (12) and said tubular member (10) in locking relationship.

10. Apparatus as claimed in claim 9, including sealing means (42) arranged to be disposed between said tubular member (10) and said elongate tube (12) at the lower end of said shank.

11. Apparatus as claimed in claim 9 or 10, including normally closed valve means (60,62) disposed between said cap and said distal end of said tubular member (10) and operable externally of the body for opening said valve means and flowing fluid into said longitudinal passageway.

12. Apparatus as claimed in any preceding claim, including cap means having an upper member (132) and a lower member (140) in rotatable relationship, said lower member being affixed to the proximal end of said tubular member (10), a cam member (136) having radially extending lobes and having a central passage adapted to fit over the proximal end of said elongate tube (12) when supported internally of said upper member, said lower member having a central opening (138) in cooperating relationship relative to said cam and having detents for receiving said lobes, said upper member and said lower member defining handle means so that engagement by an operator causes said upper member to rotate relative to said lower member for placing said lobes into said detents for locking said elongate tube to said tubular member.

13. Apparatus for cleaning the lens (53) of a viewing camera used in biological observation by penetration into a cavity of a living being, the camera having an elongate tube (12) housing fibre optics for producing a lighted area from a light source and an image relay for a television camera, with a lens (52) at the distal end of said elongate tube, the apparatus comprising:
a tubular member (10) concentrically mounted around said elongate tube and being spaced therefrom to form a longitudinal passageway (22) for cleaning fluid to flow to said lens,
means (63) for generating a vortex of said fluid disposed at the end of said longitudinal passageway, said means for generating a vortex including a plurality of vanes (64) circumferentially spaced about an axis (CL) that is arranged to be disposed coaxially relative to the longitudinal axis of said lens, and
means (60,62) for admitting fluid into said longitudinal passageway whereby the flow of fluid formed into a vortex may be directed over the lens on demand of an operator.
